(19) Europäisches Patentamt
European Patent Office
Office européen des brevets

(11) EP 1 852 118 A1

(12) **EUROPEAN PATENT APPLICATION**
published in accordance with Art. 158(3) EPC

(43) Date of publication:
07.11.2007 Bulletin 2007/45

(51) Int Cl.:
*A61K 31/4965* (2006.01)   *A61K 9/18* (2006.01)
*A61K 47/02* (2006.01)   *A61K 47/04* (2006.01)
*A61P 9/10* (2006.01)   *A61P 29/00* (2006.01)
*A61P 37/00* (2006.01)   *A61P 43/00* (2006.01)

(21) Application number: 06712277.0

(22) Date of filing: 24.01.2006

(86) International application number:
PCT/JP2006/301061

(87) International publication number:
WO 2006/080312 (03.08.2006 Gazette 2006/31)

(84) Designated Contracting States:
AT BE BG CH CY CZ DE DK EE ES FI FR GB GR
HU IE IS IT LI LT LU LV MC NL PL PT RO SE SI
SK TR

(30) Priority: 25.01.2005 JP 2005016308

(71) Applicant: Kowa Company, Ltd.
Naka-ku
Nagoya-shi, Aichi-ken 460-8625 (JP)

(72) Inventors:
• MIURA, Hiroshi
2-1, Ohnoshinden, Fuji-shi, Shizuoka, 417-8650
(JP)
• HARUTA, Sumie
Taisho Phamaceutical Co.,Ltd
Kita-ku,Saitama-shi,Saitama 331-0811 (JP)
• TAKEUCHI, Hirofumi
Gifu-shi, Gifu, 502-0006 (JP)

(74) Representative: Hartz, Nikolai
Wächtershauser & Hartz
Weinstrasse 8
80333 München (DE)

(54) **METHOD FOR PRODUCING ADSORPTIVE POROUS BODY**

(57) The present invention relates to a method for producing a 2-benzyl-5-(4-chlorophenyl)-6-[4-(methylthio)phenyl]-2H-pyridazin-3-one adsorptive porous material **characterized by** treating a porous material with a halogen-containing organic solvent in which 2-benzyl-5-(4-chlorophenyl)-6-[4-(methylthio)phenyl]-2H-pyridazin-3-one is dissolved. The production method of the present invention is simple and easy, and the obtained adsorptive porous material is excellent in dissolution properties of 2-benzyl-5-(4-chlorophenyl)-6-[4-(methylthio)phenyl]-2H-pyridazin-3-one.

EP 1 852 118 A1

**Description**

Technical Field

The present invention relates to a method for producing 2-benzyl-5-(4-chlorophenyl)-6-[4-(methylthio)phenyl]-2H-pyridazin-3-one adsorptive porous material excellent in dissolution properties therefrom.

Background Art

It is known that 2-benzyl-5-(4-chlorophenyl)-6-[4-(methylthio)phenyl]-2H-pyridazin-3-one has an excellent interleukin-1β production suppressive effect and that it is useful as a preventive and therapeutic drug for immune system diseases, inflammatory diseases and ischemic diseases (Patent Document 1). However, this compound is extremely insoluble in water, and poorly dissoluble from a preparation which contains the compound, and therefore improvement in dissolution properties thereof has been demanded.

As methods for improving dissolution properties of a poorly water-soluble drug, techniques of micronization of the drug or preparation of derivatives thereof have been known. As for 2-benzyl-5-(4-chlorophenyl)-6-[4-(methylthio)phenyl]-2H-pyridazin-3-one, however, dissolution properties cannot be improved by micronization of the compound and the medicinal effect thereof changes when converted into derivatives, which is not preferable.

As a method for improving dissolution properties of a medicinal agent from a preparation, a method of treating a composition containing an extremely poorly water-soluble drug such as 2-benzyl-5-(4-chlorophenyl)-6-[4-(methylthio)phenyl]-2H-pyridazin-3-one and a porous material with a supercritical fluid or a subcritical fluid of carbon dioxide is also known (Patent Document 2) but the method is not preferable in that it needs to use pressure resistant container and apparatus in order to bring carbon dioxide to supercritical or subcritical state, which also makes the production process complicated.

[Patent Document 1] Japanese Patent Laid-Open No. 2000-198776
[Patent Document 2] WO2004/096280

DISCLOSURE OF THE INVENTION

PROBLEMS TO BE SOLVED BY THE INVENTION

An object of the present invention is to provide a simple and easy method for producing 2-benzyl-5-(4-chlorophenyl)-6-[4-(methylthio)phenyl]-2H-pyridazin-3-one preparations excellent in dissolution properties therefrom.

MEANS FOR SOLVING THE PROBLEMS

The present inventors have conducted intensive studies in search for a simple and easy method for producing a preparation which comprises 2-benzyl-5-(4-chlorophenyl)-6-[4-(methylthio)phenyl]-2H-pyridazin-3-one which is excellent in dissolution properties, and consequently found that when a porous material is treated with a solution of 2-benzyl-5-(4-chlorophenyl)-6-[4-(methylthio)phenyl]-2H-pyridazin-3-one dissolved in a halogen-containing organic solvent, 2-benzyl-5-(4-chlorophenyl)-6-[4-(methylthio)phenyl]-2H-pyridazin-3-one adsorbs to the porous material and that the dissolution properties therefrom are excellent, and thus completed the present invention.

That is, a method for producing a 2-benzyl-5-(4-chlorophenyl)-6-[4-(methylthio)phenyl]-2H-pyridazin-3-one adsorptive porous material comprising treating a porous material with a solution of 2-benzyl-5-(4-chlorophenyl)-6-[4-(methylthio)phenyl]-2H-pyridazin-3-one dissolved in a halogen-containing organic solvent is provided.

In addition, the present invention provides a 2-benzyl-5-(4-chlorophenyl)-6-[4-(methylthio)phenyl]-2H-pyridazin-3-one adsorptive porous material produced by the above production method and a pharmaceutical composition containing the same.

Furthermore, the present invention provides a method for improving dissolution properties of 2-benzyl-5-(4-chlorophenyl)-6-[4-(methylthio)phenyl]-2H-pyridazin-3-one comprising dissolving 2-benzyl-5-(4-chlorophenyl)-6-[4-(methylthio)phenyl]-2H-pyridazin-3-one in a halogen-containing organic solvent and treating a porous material with the solution.

EFFECT OF THE INVENTION

According to the present invention, 2-benzyl-5-(4-chlorophenyl)-6-[4-(methylthio)phenyl]-2H-pyridazin-3-one adsorptive porous material can be produced simply and easily and the provided adsorptive porous material is excellent in dissolution properties of 2-benzyl-5-(4-chlorophenyl)-6-[4-(methylthio)phenyl]-2H-pyridazin-3-one.

Best Mode for Carrying Out the Invention

A content ratio of 2-benzyl-5-(4-chlorophenyl)-6-[4-(methylthio)phenyl]-2H-pyridazin-3-one (hereinbelow also referred to as Compound A) in a 2-benzyl-5-(4-chlorophenyl)-6-[4-(methylthio)phenyl]-2H-pyridazin-3-one adsorptive porous material (hereinbelow also referred to as the adsorptive porous material of the present invention) is preferably 0.1 to 91 mass%, more preferably 1 to 67 mass%, and particularly preferably 2 to 50 mass%.

Examples of the porous material to be used in the present invention include carbonaceous porous material, aluminous porous material, silicic porous material and the like. The porous material may be contained preferably in a ratio of 9 to 99.9 mass%, more preferably 33 to 99 mass%, and particularly preferably 50 to 98 mass% in the adsorptive porous material of the present invention.

Examples of the carbonaceous porous material include powdery activated carbon, granular activated carbon, molecular sieve charcoal, beads-shaped activated carbon, fibrous activated carbon, large surface area activated carbon, molded activated carbon and honeycomb activated carbon and the like.

Examples of the aluminous porous material include alumina, aluminium oxide, activated alumina, boehmite gel and zeolite and the like.

Examples of the silicic porous material include light anhydrous silicic acid, hydrated silicon dioxide, silicon dioxide, magnesium aluminosilicate, calcium silicate, magnesium silicate, magnesium aluminum silicate, light anhydrous silicic acid-containing hydroxypropylcellulose, diatom soil, synthetic aluminum silicate, synthetic aluminum silicate/hydroxypropyl starch/crystalline cellulose, synthetic magnesium sodium silicate, colloidal hydrous aluminum silicate and zeolite.

Silicic porous material is preferable as the porous material to be used in the present invention. Furthermore, light anhydrous silicic acid, hydrated silicon dioxide, silicon dioxide, calcium silicate are preferable as the silicic porous material. Specific examples thereof include commercial products such as Sylysia 250, Sylysia 320, Sylysia 350, Sylysia 530, Sylysia 740 (produced by Fuji Silysia Chemical, Ltd.), Adsolider 101, Adsolider 102 (produced by Freund Corp.), Carplex # 67 (produced by Shionogi & Co., Ltd.), Aerosil 200, Aerosil 300 (produced by Nippon Aerosil Co., Ltd.), Sunsphere H-51 (Asahi Glass Co., Ltd.) and Flow Light RE (produced by Eisai Co., Ltd.) and the like.

An average pore diameter of the porous material to be used in the present invention is preferably 1 to 1000 nm, more preferably 2 to 500nm, and particularly preferably 2 to 200 nm. The average pore diameter can be measured, for example, by gas adsorption method.

A specific surface area of the porous material to be used in the present invention is preferably 1 to 2000 $m^2/g$, more preferably 100 to 1800 $m^2/g$, and particularly preferably 200 to 1500 $m^2/g$. The specific surface area can be measured, for example, by gas adsorption method.

A mass ratio of Compound A to the porous material to be used in the present invention is preferably 1:0.1 to 1000, more preferably 1:0.5 to 100 and particularly preferably 1:1 to 50.

Examples of a halogen-containing organic solvent to be used in the present invention include dichloromethane, chloroform, carbon tetrachloride and 1,2-dichloroethane, among which dichloromethane is particularly preferable. These halogen-containing organic solvents can be used singly or in combination. Dissolution properties of Compound A from the adsorptive porous material are improved by treating the porous material with a solution of Compound A dissolved in a halogen-containing organic solvent to prepare the adsorptive porous material of the present invention.

A mass ratio of Compound A to the halogen-containing organic solvent to be used in the present invention is preferably 1:0.1 to 1000000, more preferably 1:5 to 100000, and particularly preferably 1:10 to 50000.

A treating time with a solution of Compound A dissolved in a halogen-containing organic solvent is preferably 0.1 minute to 24 hours, more preferably 0.5 minute to 12 hours, and particularly preferably 1 minute to 8 hours. A treating temperature is preferably -100 to 85°C, more preferably 0 to 65°C, and particularly preferably 10 to 40°C .

A production process of treating the porous material with a solution of Compound A dissolved in a halogen-containing organic solvent is not particularly limited but examples thereof include (1) a process comprising adding a halogen-containing organic solvent to Compound A, adding a porous material to the solution in which Compound A is dissolved completely, stirring the mixture to obtain a suspension, and then removing the halogen-containing organic solvent by evaporation under reduced pressure, distillation under normal pressure, distillation under reduced pressure, drying by naturally evaporating the solvent, warming drying, lyophilization, filtering, centrifugal separation, etc., and subsequently drying the residue to collect the adsorptive porous material; (2) a process comprising adding a halogen-containing organic solvent to Compound A, adding a porous material to the solution in which Compound A is dissolved completely, stirring the mixture to obtain a suspension, and then spray-drying the suspension to collect the adsorptive porous material.

The thus produced adsorptive porous material of the present invention ordinarily has an average particle diameter preferably of 1 $\mu$m or more, more preferably 1 to 2000 $\mu$m, and particularly preferably 3 to 500 $\mu$m. The average particle diameter can be measured by laser diffraction method, etc.

When the porous material is treated with a solution of Compound A dissolved in a halogen-containing organic solvent, various ingredients acceptable as additives in pharmaceutical products can be added as desired unless they do not hinder the effects of the present invention, and examples thereof include polymer compounds and surfactants.

Examples of polymer compound include pullulan, sodium carboxymethylcellulose, sodium alginate, xanthan gum, polyvinylpyrrolidone, carboxyvinyl polymer, methyl cellulose, hydroxypropyl methylcellulose, carrageenan, agar and gelatine .

Examples of surfactant include nonionic surfactants such as polyoxyethylene polyoxypropylene glycol, polyoxyethylene hydrogenated castor oil, polyoxyethylene alkyl ethers such as polyoxyethylene lauryl ether, polyoxyethylene sorbitan fatty acid ester (polysorbate) and sorbitan fatty acid ester such as sorbitan monostearate, cationic surfactants such as benzalkonium chloride and benzethonium chloride, cetylpyridinium chloride, anionic surfactants such as calcium stearate, magnesium stearate and sodium lauryl sulfate.

The adsorptive porous material of the present invention can be used as is as a pharmaceutical composition, but additives ordinarily used in pharmaceutical compositions can be added to prepare an oral pharmaceutical composition and a non-oral pharmaceutical composition. A content of adsorptive porous material of the present invention is preferably 0.1 to 100 mass%, more preferably 0.1 to 90 mass%, and particularly preferably 0.1 to 80 mass% in the pharmaceutical composition.

Examples of additives for oral pharmaceutical compositions include excipients such as lactose, crystalline cellulose, refined sugar, mannitol, light anhydrous silicic acid and calcium hydrogenphosphate; binders such as methyl cellulose, hydroxypropylcellulose, hydroxypropyl methylcellulose, gelatine, polyvinylpyrrolidone and pullulan; disintegrators such as croscarmellose sodium, carmellose calcium, crospovidone and low-substituted hydroxypropylcellulose; lubricants such as magnesium stearate and talc; coloring agents such as tar dye, iron sesquioxide; corrigents such as stevia, aspartame and flavor.

Examples of additives for non-oral pharmaceutical compositions include solvents such as monohydric alcohols such as benzyl alcohol, polyhydric alcohols such as concentrated glycerin and 1,3-butylene glycol, esters such as diisopropyl adipate, triacetin and isopropyl myristate, ketones such as crotamiton, oils and fats such as oleic acid and castor oil; water-soluble polymers such as celluloses such as hydroxyethyl cellulose and hydroxypropylcellulose, refined sugar, polysaccharides such as β-cyclodextrin, sugar alcohols such as sorbitol and mannitol, synthetic polymers such as carboxyvinyl polymer, gelatine, starch, polyvinyl alcohol, polyvinylpyrrolidone, polyacrylic acid, sodium polyacrylate; surfactants such as anionic surfactant such as calcium stearate, magnesium stearate and sodium lauryl sulfate, cationic surfactants such as benzalkonium chloride, benzethonium chloride and cetylpyridinium chloride, nonionic surfactants such as glyceryl monostearate, sucrose fatty acid ester, polyoxyethylene hydrogenated castor oil, polyoxyethylene sorbitan fatty acid ester; sorbefacient such as terpenes such as L-menthol and dL-camphor and higher fatty acids such as oleic acid; stabilizing agents such as phenolic substances such as methyl parahydroxybenzoate and propyl parahydroxybenzoate, neutral substances such as chlorobutanol and phenylethyl alcohol, inverted soaps such as benzalkonium chloride and benzethonium chloride, anti-oxidants such as vitamin E and butylhydroxyanisole, reducing agents such as ascorbic acid, sodium bisulfite and sodium thiosulfate, chelating reagents such as citric acid or tartaric acid and the salts thereof, lecithin, ethylenediaminetetraacetic acid (edetic acid); pH adjusting agents such as phosphoric acid, acetic acid, boric acid, succinic acid, phthalic acid and the salts thereof, glycine, and sodium hydroxide.

Examples of a form of the pharmaceutical composition of the present invention include oral preparations such as tablets, capsules, granules and fine grain agents and non-oral preparations such as injection agents, suppositories, vaginal agents, sublingual agents, implant agents, eye drops and spray agents.

Examples

The present invention is described more specifically by Examples and Comparative Examples but the present invention is not limited to these Examples.

Example 1

To a solution prepared by completely dissolving 150 mg of Compound A in 100 mL of dichloromethane, 1500 mg of light anhydrous silicic acid (Sylysia 350, average particle diameter: 4 μm, average pore diameter: 21 nm, specific surface area: 300 $m^2$/g; produced by Fuji Silysia Chemical Co., Ltd.) was added, and this mixture was stirred (temperature: 25°C, time: 10 minutes) to obtain a suspension. Then dichloromethane was removed under reduced pressure from the suspension with an evaporator and residual substances were dried by a vacuum dryer and Adsorptive Porous Material 1 of the present invention was obtained.

Comparative Examples 1 to 3

Adsorptive Porous Materials 1 to 3 of Comparative Examples were obtained in the same manner as in Example 1 except that dichloromethane was replaced with acetone (Comparative Example 1), ethanol (Comparative Example 2) and acetone/ethanol mixed solution (1:1) (Comparative Example 3) .

<Dissolution Test>

Dissolution Test was performed on Adsorptive Porous Material 1 of the present invention and Adsorptive Porous Materials 1 to 3 of Comparative Examples. The dissolution test was performed following the second test method (paddle method) of the general test method of Japanese Pharmacopoeia. Fifty-five mg of the adsorptive porous material was put into 900 mL of a test liquid (0.3 mass% sodium lauryl sulfate aqueous solution) under the conditions of $37\pm1$°C (temperature) and 50 r/min (paddle rotation rate) . An amount of Compound A dissolved into the test liquid after 5, 30, 60, 120 minutes was quantified by liquid chromatograph using a reversed-phase column (Inertsil ODS-2, produced by GL Sciences Inc.) and a dissolution ratio (%) for 5 mg of Compound A was calculated.
The results are shown in Table 1.
[Table 1]

| | | Example | Comparative Example | | |
|---|---|---|---|---|---|
| | | 1 | 1 | 2 | 3 |
| Compound A mg | | 150 | 150 | 150 | 150 |
| Light anhydrous silicic acid (Sylysia 350) mg | | 1500 | 1500. | 1500 | 1500 |
| Dichloromethane mL | | 100 | - | - | - |
| Acetone mL | | - | 100 | - | - |
| Ethanol mL | | - | - | 100 | - |
| Acetone/ethanol mixed solution (1:1) mL | | - | - | - | 100 |
| Dissolution Ratio (%) | Stirring time | | | | |
| | 5 minutes | 92.3 | 18.4 | 8.0 | 7.9 |
| | 30 minutes | 96.1 | 19.8 | 9.9 | 10.9 |
| | 60 minutes | 97.1 | 20.9 | 12.7 | 13.8 |
| | 120 minutes | 97.1 | 23.2 | 17.0 | 18.6 |

The dissolution properties of Compound A have been improved dramatically in Adsorptive Porous Material 1 of the present invention (Example 1). On the other hand, Compound A hardly dissolved from the adsorptive porous materials produced by using acetone (Comparative Example 1), ethanol (Comparative Example 2) or acetone/ethanol mixed solution (1:1) (Comparative Example 3) in place of dichloromethane.

<Adsorption Test to Porous Material>

To a solution prepared by completely dissolving 1.5 mg of Compound A in 1.5 mL of dichloromethane, 15 mg of porous material (light anhydrous silicic acid (Sylysia 350, produced by Fuji Silysia Chemical Co., Ltd.) or silicon dioxide (Sylysia 530, produced by Fuji Silysia Chemical Co., Ltd.) was dispersed, and this mixture was shaken for two hours using Roter RT-50 (TAITEC) and an amount of Compound A which adsorbed simply to the light anhydrous silicic acid or silicon dioxide ($\mu$g/mg porous material) was calculated by measuring the absorbance (280 nm) of a filtrate obtained by filtering with a membrane filter (0.45 $\mu$m PTFE). The amount of Compound A which adsorbed simply to the light anhydrous silicic acid or silicon dioxide ($\mu$g/mg porous material) was calculated by the following computational expression.
Concentration of Compound A in the filtrate ($\mu$g/mL)

= ABS ÷ Slope of analytical curve × Dilution ratio

Here, ABS represents the absorbance.

$$\text{Amount of adsorbed Compound A } (\mu g/mg \text{ porous}$$
$$\text{material}) = (\text{Concentration of Compound A before the}$$
$$\text{porous material was dispersed } (\mu g/mL) - \text{Concentration of}$$
$$\text{Compound A in the filtrate } (\mu g/mL)) \times \text{Volume of Compound}$$
$$\text{A solution } (mL) \div \text{Mass of porous material } (mg)$$

Calculation was also conducted using ethanol in place of dichloromethane as a comparison. The results are shown in Table 2.

[Table 2]

|  | (μg/mg porous material) | |
| --- | --- | --- |
|  | Dichloromethane | Ethanol |
| Light anhydrous silicic acid (Sylysia 350) | 17.6 | 0.0 |
| Silicon dioxide (Sylysia 530) | 24.8 | 0.0 |

As a result, when dichloromethane was used, Compound A adsorbed to the light anhydrous silicic acid and the silicon dioxide, both of which were porous materials, whereas when ethanol was used, Compound A did not adsorb to the light anhydrous silicic acid and the silicon dioxide. In addition, when dichloromethane was used, it was found that Compound A existed in amorphous state as a result of differential scanning calorimetry analysis.

Production Example 1

150 g of the adsorptive porous material of the present invention produced in Example 1 was subjected to size regulation with New Speed mill (ND-02, produced by Okada Seiko Co., Ltd.) equipped with a screen of 1 mmφ diameter. After 110 g of the size regulated adsorptive porous material of the present invention, 42 g of lactose (100 mesh lactose, produced by DMV Corporation), 100 g of crystalline cellulose (Avicel PH-102, produced by Asahi Kasei Corporation) and 45 g of low-substituted hydroxypropylcellulose (L-HPC (LH-11), produced by Shin-Etsu Chemical Co., Ltd.) were mixed with a V type mixer for 10 minutes, 3 g of magnesium stearate was added to this mixture and they were further mixed for 5 minutes. This mixture was made into tablets with a tablet machine (AP-38, produced by Hata Iron works Co., Ltd.) and tablets of 300 mg per tablet (containing 10 mg of Compound A) were produced.

**Claims**

1.   A method for producing a 2-benzyl-5-(4-chlorophenyl)-6-[4-(methylthio)phenyl]-2H-pyridazin-3-one adsorptive porous material comprising treating a porous material with a solution of 2-benzyl-5-(4-chlorophenyl)-6-[4-(methylthio) phenyl]-2H-pyridazin-3-one dissolved in a halogen-containing organic solvent.

2.   The method for producing an adsorptive porous material according to claim 1, wherein the porous material is a carbonaceous porous material, an aluminous porous material or a silicic porous material.

3.   The method for producing an adsorptive porous material according to claim 1, wherein the porous material is a silicic porous material.

4.   The method for producing an adsorptive porous material according to claim 3, wherein the silicic porous material is light anhydrous silicic acid, hydrated silicon dioxide, silicon dioxide or calcium silicate.

5.   The method for producing an adsorptive porous material according to any one of claims 1 to 4, wherein the halogen-containing organic solvent is one or more selected from dichloromethane, chloroform, carbon tetrachloride and 1,2-dichloroethane.

6. The method for producing an adsorptive porous material according to claim 5, wherein the halogen-containing organic solvent is dichloromethane.

7. A 2-benzyl-5-(4-chlorophenyl)-6-[4-(methylthio)phenyl]-2H-pyridazin-3-one adsorptive porous material produced by the method for producing an adsorptive porous material according to any one of claims 1 to 6.

8. A pharmaceutical composition comprising the 2-benzyl-5-(4-chlorophenyl)-6-[4-(methylthio)phenyl]-2H-pyridazin-3-one adsorptive porous material according to claim 7.

9. A method for improving dissolution properties of 2-benzyl-5-(4-chlorophenyl)-6-[4-(methylthio)phenyl]-2H-pyridazin-3-one comprising dissolving 2-benzyl-5-(4-chlorophenyl)-6-[4-(methylthio)phenyl]-2H-pyridazin-3-one in a halogen-containing organic solvent and treating a porous material with the solution.

## INTERNATIONAL SEARCH REPORT

| International application No. |
| --- |
| PCT/JP2006/301061 |

**A. CLASSIFICATION OF SUBJECT MATTER**
*A61K31/4965*(2006.01), *A61K9/18*(2006.01), *A61K47/02*(2006.01), *A61K47/04*
(2006.01), *A61P9/10*(2006.01), *A61P29/00*(2006.01), *A61P37/00*(2006.01),
*A61P43/00*(2006.01)

According to International Patent Classification (IPC) or to both national classification and IPC

**B. FIELDS SEARCHED**

Minimum documentation searched (classification system followed by classification symbols)
A61K9/18, A61K31/4965, A61K47/02, A61K47/04

Documentation searched other than minimum documentation to the extent that such documents are included in the fields searched

| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
REGISTRY(STN), CAplus(STN)

**C. DOCUMENTS CONSIDERED TO BE RELEVANT**

| Category* | Citation of document, with indication, where appropriate, of the relevant passages | Relevant to claim No. |
| --- | --- | --- |
| Y | WO 2004/096280 A1 (Kowa Co., Ltd.), | 1-5,7-9 |
| A | 11 November, 2004 (11.11.04), | 6 |
| | Full text; particularly, 'Background Art'; | |
| | Claims | |
| | & EP 1618895 A1 | |
| | | |
| Y | WO 2004/096281 A1 (Kowa Co., Ltd.), | 1-5,7-9 |
| A | 11 November, 2004 (11.11.04), | 6 |
| | Full text; particularly, 'Background Art'; | |
| | Claims | |
| | (Family: none) | |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 05 April, 2006 (05.04.06) | 18 April, 2006 (18.04.06) |

| Name and mailing address of the ISA/ | Authorized officer |
| --- | --- |
| Japanese Patent Office | |
| Facsimile No. | Telephone No. |

Form PCT/ISA/210 (second sheet) (April 2005)

**INTERNATIONAL SEARCH REPORT**

International application No.

PCT/JP2006/301061

C (Continuation).  DOCUMENTS CONSIDERED TO BE RELEVANT

| Category* | Citation of document, with indication, where appropriate, of the relevant passages | Relevant to claim No. |
|---|---|---|
| Y<br>A | JP 63-119426 A  (Mitsubishi Chemical Industries Ltd.),<br>24 May, 1988 (24.05.88),<br>Full text; particularly, page 2, upper left column, line 4 to upper right column, line 19; examples<br>& WO 88/03023 A1 | 1-5,7-9<br>6 |
| Y<br>A | JP 3-109332 A  (Shiseido Co., Ltd.),<br>09 May, 1991 (09.05.91),<br>Full text; particularly, Claims; page 3, upper right column, lines 11 to 14<br>(Family: none) | 1-5,7-9<br>6 |
| Y<br>A | JP 2005-501820 A  (Pfizer Products Inc.),<br>20 January, 2005 (20.01.05),<br>Full text; particularly, Claims; Par. Nos. [0007], [0307] to [0313]<br>& WO 2003/000238 A1      & EP 1404302 A1<br>& US 2003/054037 A1      & CN 1523979 A<br>& KR 2004023632 A | 1-5,7-9<br>6 |
| A | WO 2003/086405 A1  (Kowa Co., Ltd.),<br>23 October, 2003 (23.10.03),<br>Full text<br>& EP 1495759 A1          & US 2004/019122 A1<br>& CN 1646128 A          & CA 2481595 A | 1-9 |
| A | JP 2004-131393 A  (Kowa Co., Ltd.),<br>30 April, 2004 (30.04.04),<br>Full text<br>(Family: none) | 1-9 |
| A | JP 2004-067533 A  (Kowa Co., Ltd.),<br>04 March, 2004 (04.03.04),<br>Full text<br>(Family: none) | 1-9 |

Form PCT/ISA/210 (continuation of second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000198776 A

- WO 2004096280 A